# EUROPEAN PATENT SPECIFICATION

(11) **EP 0 352 547 B1**
(45) Date of publication and mention of the grant of the patent: **02.11.1994**
(21) Application number: 89112729.2
(22) Date of filing: 12.07.1989
(51) Int. Cl.: C12Q 1/32, C12Q 1/37

(54) **LDH1 assay**
Nachweis von LDH1
Essai de LDH1

(30) Priority: 15.07.1988 JP 177579/88
(43) Date of publication of application: 31.01.1990
(73) Proprietor: INTERNATIONAL REAGENTS CORPORATION, Kobe-shi Hyogo 651 (JP)
(72) Inventor: Shirahase, Yasushi, Kobe-shi Hyogo-ken (JP); Isshiki, Kenji, Kobe-shi Hyogo-ken (JP); Watazu, Yoshifumi, Akashi-shi Hyogo-ken (JP)
(74) Representative: VOSSIUS & PARTNER

(56) References cited:
- EP-A- 0 292 838
- DE-A- 2 513 407
- FR-A- 2 418 464
- FR-A- 2 603 702
- PATENT ABSTRACTS OF JAPAN, vol. 12, no. 172 (C-497)(3019), 21 May 1988#
- PATENT ABSTRACTS OF JAPAN, vol. 9, no. 309 (C-318)(2032), 05 December 1985#
- PATENT ABSTRACTS OF JAPAN, vol. 13, no. 39 (C-563)(3387), 27 January 1989#

## Description

The present invention relates to a process for the determination of an LDH₁ fraction in a sample.

LDH (lactate dehydrogenase) has five isozymes, that is LDH₁ through LDH₅. Each organ has its own composition of these isozymes. For example, LDH₁ is present in the myocardium in the largest amount. Since LDH₁ escapes from the myocardium into the blood during myocardial infarction, the increase of the LDH₁ serum level can be used for the diagnosis of such a disease. Therefore, an LDH₁ isozyme assay is clinically significant.

In the most common LDH isozyme assay, LDH₁, LDH₂, LDH₃, LDH₄ and then LDH₅ are fractionated in the order of electrophoretic mobility. An immunological LDS assay is also known. In another LDH assay disclosed in Japanese Patent Kokai Publication No. 6477/1983, a coenzyme derivative is used. In addition, LDH assays wherein a sample is treated under alkaline conditions are described in Japanese Patent Publication No. 28280/1985 and Japanese Patent Kokai Publication No. 278997/1987.

The above-mentioned electrophoretic or immunological assay is unsuitable for clinical autoanalysis because of the complicated process and a long operation time. In addition, the LDH isozymes may be insufficiently fractionated by the electrophoretic method.

Strictly speaking, it is impossible to assay the LDH isozyme fractions by the method wherein the coenzyme derivative is used, while the ratio of the subunit to the M subunit in the enzyme can be suitably determined by the method.

In the method comprising the alkaline treatment of the sample, more than 50 % of LDH₁ may be inactivated during the inhibition of the other isozymes. In addition, the process is difficult and time-consuming.

Thus, the above-mentioned conventional LDH assays are unsatisfactory for clinical examination and diagnosis.

Thus, an object of the present invention is to provide an LDH₁ assay useful for the simple, fast and reliable clinical examination or diagnosis including autoanalysis.

Accordingly, the present invention provides a process for the determination of an LDH₁ fraction in a sample, which comprises inhibiting LDH₂, LDH₃, LDH₄ and LDH₅ in the sample with a protease in the presence of a protein-denaturating agent and then determining the remaining uninhibited LDH₁.

The present process is suitable for the determination of the LDH₁ fraction in a clinical sample such as serum or plasma.

The protease used in the present invention may be any protease suitable for the purpose of the invention. For example, α-chymotrypsin, trypsin, thrombin, elastase, endoprotease, subtilisin, bromelain, papain, carboxypeptidase, pronase, pepsin or cathepsin can be used. The proteases disclosed in Japanese Patent Kokai Publication No. 149399/1985 may also be used.

Although the amount of the protease is not critical in the present invention and varies with other assay conditions, α-chymotrypsin, for example, can be used in a concentration in the range of from 10 to 1,000 units/ml.

In the present invention, any protein-denaturating agent having an activity suitable for the process can be used. For example, cholic acid, deoxycholic acid, taurocholic acid, taurodeoxycholic acid, guanidine (hydrochloride), trichloroacetic acid, urea, thiourea or thiocyanate are preferably used as the protein-denaturating agent as such or in the form of an admixture.

Although the amount of the protein-denaturating agent is not critical and varies with other assay conditions, for example 0.05 to 5 M guanidine can be used.

The LDH₁ isozyme remaining uninhibited can be determined according to an enzyme assay selected from a lot of conventional assays widely used in clinical examinations. Such an assay used in the present process should be easily carried out within a short time with high precision, sensitivity and accuracy.

In a preferred embodiment of the present process, the LDH₁ isozyme remaining uninhibited is determined by an enzyme assay which comprises catalytically developing a chromogen or dye precursor with the isozyme and then measuring absorption in the visible light range. It is also possible to measure ultraviolet absorption of coenzyme NADH reduced by a catalytic effect of the LDH₁ isozyme.

In another preferred embodiment, the LDH₁ isozyme is determined according to the Wroblewski method which is a well-known enzyme assay performed under neutral conditions. It is found that the inhibition of the isozymes LDH₂₋₅ can be effected in a wide pH range according to the present invention (cf. Example 1). Therefore, when the determination of the LDH₁ isozyme remaining uninhibited is performed under a neutral condition, the present assay can be advantageously carried out under the same neutral condition throughout the assay, while complicated pH correction is necessary in the above-mentioned conventional assay comprising the alkaline treatment of the sample.

The present invention is further illustrated by the following Examples. However, the invention is not limited to these Examples.

### Example 1

To an authentic sample (0.04 ml) of each of the isozymes LDH₁₋₅, which was obtained by purifying human blood, a buffer (0.2 ml) containing α-chymotrypsin (450 units/ml) and 0.65 M guanidine was added and it was incubated at 37°C for 5 minutes. The buffers used and the pH values thereof were as shown in the following Table 1.

Then, after the addition of a 0.1 M Tris buffer (2.5 ml) containing 2 mM sodium pyruvate and 0.2 mM NADH, by which the pH value was regulated at 7.8, the changing rate of the absorbance per minute was measured at 340 nm. Residual activity of each isozyme was calculated from the measured value. The results are shown in the following Table 1.

It was found that only the LDH₁ fraction among the isozymes LDH₁₋₅ could be determined by the present assay and that the LDH₁ isozyme remained uninhibited at any pH value during the inhibition of the other isozymes.

Therefore, when the LDH₁ isozyme remaining uninhibited is determined under neutral conditions, the present assay can be advantageously carried out under these neutral conditions throughout the assay without complicated pH correction.

**Table 1**

| pH (Buffer) | Residual activity (%) of | | | | |
|---|---|---|---|---|---|
| | LDH₁ | LDH₂ | LDH₃ | LDH₄ | LDH₅ |
| 6 (MES) | 83 | 1 | 1 | 0 | 0 |
| 7 (PIPES) | 85 | 2 | 0 | 1 | 0 |
| 8 (Tris) | 83 | 2 | 1 | 0 | 0 |
| 9 (CHES) | 80 | 1 | 0 | 0 | 0 |
| 10 (CHES) | 74 | 1 | 0 | 0 | 0 |
| 11 (CAPS) | 69 | 0 | 0 | 0 | 0 |

### Example 2

To the same sample (0.04 ml) as used in Example 1, a 0.1 M Tris buffer (pH 7.8; 0.2 ml) containing subtilisin (5 units/ml) and sodium deoxycholate (1.5 %) was added and incubated at 37 °C for 5 minutes. Then, after the addition of a 0.1 M Tris buffer (pH 7.8; 2.5 ml) containing 2 mM sodium pyruvate and 0.2 mM NADH, the residual activity of each isozyme was determined in the same way as in Example 1. The results are shown in Table 2.

It was found that the present LDH₁ assay could be effectively carried out at pH 7.8.

**Table 2**

| Residual activity (%) of | | | | |
|---|---|---|---|---|
| LDH₁ | LDH₂ | LDH₃ | LDH₄ | LDH₅ |
| 83 | 0 | 0 | 0 | 0 |

### Example 3

As the sample, 15 human sera (each 0.04 ml) were used. The total LDH activity of each sample was known as shown in the following Table 3.

In the same way as in Example 1, the changing rate of the absorbance per minute was measured. Then, the LDH₁ activity shown in Table 3 was determined with using a calibration curve.

Separately, the sera were subjected to a conventional immunological assay for the LDH₁ isozyme (with using Isomune-LD; Rosh). The results are also shown in Table 3.

The results of the present process well corresponded with those of the conventional immunological assay.

**Table 3**

| Sample | Total LDH activity (units/l) | LDH₁ activity (units/l) determined by | |
|---|---|---|---|
| | | the present process | the conventional immunological assay |
| 1 | 386 | 84 | 87 |
| 2 | 290 | 85 | 89 |
| 3 | 305 | 89 | 94 |
| 4 | 242 | 45 | 44 |
| 5 | 342 | 113 | 118 |
| 6 | 2147 | 1113 | 1138 |
| 7 | 239 | 73 | 69 |
| 8 | 623 | 181 | 181 |
| 9 | 387 | 70 | 74 |
| 10 | 423 | 113 | 109 |
| 11 | 1812 | 500 | 483 |
| 12 | 322 | 79 | 73 |
| 13 | 906 | 247 | 262 |
| 14 | 485 | 117 | 115 |
| 15 | 1011 | 288 | 287 |

### Example 4

To the same sample (10 µl) as used in Example 1, a 50 mM MES buffer (pH 6.0; 75 µl) containing 0.5 M guanidine hydrochloride, 2 % Triton X-405 and a protease shown in the following Table 4 was added. After incubating at 37 °C for 5 minutes, a 0.1 M Tris buffer (PH 8.0; 400 µl) containing 2 mM sodium pyruvate and 0.2 mM NADH was added. Then, the residual activity of each isozyme was determined in the same way as in Example 1. The results are shown in the Table 4.

It was found that the LDH₁ fraction could be determined with any protease, preferably with carboxypeptidase Y, cathepsin C or α-chymotrypsin.

**Table 4**

| | Residual activity (%) of | | | | |
|---|---|---|---|---|---|
| | LDH₁ | LDH₂ | LDH₃ | LDH₄ | LDH₅ |
| Pepsin | 41 | 22 | 5 | 0 | 0 |
| Carboxypeptidase P | 96 | 63 | 60 | 60 | 6 |
| Carboxypeptidase Y | 100 | 1 | 0 | 0 | 0 |
| Cathepsin | 80 | 2 | 1 | 1 | 1 |
| α-Chymotrypsin | 99 | 11 | 2 | 2 | 1 |

### Example 5

To the same sample (0.04 ml) as used in Example 1, a Tris buffer (pH 8.0; 0.2 ml) containing either α-chymotrypsin (450 units/ml) or 0.65 M guanidine was added and it was incubated at 37 °C for 5 minutes. Then, after the addition of a 0.1 M Tris buffer (2.5 ml) containing 2 mM sodium pyruvate and 0.2 mM NADH, by which the pH value was regulated at 7.8, the residual activity of each isozyme was determined in the same way as in Example 1. The results are shown in Table 5.

It was found that the LDH₂₋₅ fractions were insufficiently inactivated. Therefore, it is essential in the present assay to use a protease in the presence of the protein-denaturating agent.

**Table 5**

| | Residual activity (%) of | | | | |
|---|---|---|---|---|---|
| | LDH₁ | LDH₂ | LD H₃ | LD H₄ | LD H₅ |
| When guanidine was added without α-chymotrypsin | 96 | 87 | 79 | 45 | 40 |
| When α-chymotrypsin was added without guanidine | 100 | 78 | 29 | 2 | 0 |

## Claims

1. A process for the determination of an LDH₁ fraction in a sample, which comprises inhibiting LDH₂, LDH₃, LDH₄ and LDH₅ in the sample with a protease in the presence of a protein-denaturating agent and then determining the remaining uninhibited LDH₁.

2. The process of claim 1, wherein the sample is a clinical sample of serum or plasma.

3. The process of claim 1 or 2, wherein the protease is α-chymotrypsin, trypsin, thrombin, elastase, endoprotease, subtilisin, bromelain, papain, carboxypeptidase, pronase, pepsin or cathepsin.

4. The process of claim 3, wherein a-chymotrypsin is used as the protease in a concentration in the range of from 10 to 1,000 units/ml.

5. The process of any one of claims 1 to 4, wherein the protein-denaturating agent is cholic acid, deoxycholic acid, taurocholic acid, taurodeoxycholic acid, guanidine (hydrochloride), trichloroacetic acid, urea, thiourea or thiocyanate or a mixture thereof.

6. The process of claim 5, wherein 0.05 to 5 M guanidine is used as the protein-denaturating agent.

7. The process of any one of claims 1 to 6, wherein the LDH₁ isozyme remaining uninhibited is determined according to an enzyme assay which comprises catalytically developing a chromogen or dye precursor with the isozyme and then measuring the absorption in a visible light range or which comprises measuring the ultraviolet absorption of the coenzyme reduced by the catalytic activity of the isozyme.

8. The process of claim 7, which is carried out under neutral conditions throughout the assay without pH correction.

## Patentansprüche

1. Verfahren zur Bestimmung einer LDH₁ Fraktion in einer Probe, umfassend die Hemmung von LDH₂, LDH₃, LDH₄ und LDH₅ in der Probe mit einer Protease in Gegenwart eines Protein-denaturierenden Mittels und im Anschluß daran die Bestimmung des restlichen nicht-gehemmten LDH₁.

2. Verfahren nach Anspruch 1, wobei die Probe eine klinische Serum- oder Plasmaprobe ist.

3. Verfahren nach Anspruch 1 oder 2, wobei die Protease α-Chymotrypsin, Trypsin, Thrombin, Elastase, Endoprotease, Subtilisin, Bromelain, Papain, Carboxypeptidase, Pronase, Pepsin oder Cathepsin ist.

4. Verfahren nach Anspruch 3, wobei als Protease α-Chymotrypsin in einem Konzentrationsbereich von 10 bis 1000 Einheiten/ml verwendet wird.

5. Verfahren nach einem der Ansprüche 1 bis 4, wobei das Protein-denaturierende Mittel Cholsäure, Desoxycholsäure, Taurocholsäure, Taurodesoxycholsäure, Guanidin (Hydrochlorid), Trichloressigsäure, Harnstoff, Thioharnstoff oder Rhodanid oder ein Gemisch davon ist.

6. Verfahren nach Anspruch 5, wobei als Proteindenaturierendes Mittel 0,05 bis 5 M Guanidin verwendet wird.

7. Verfahren nach einem der Ansprüche 1 bis 6, wobei das restliche nicht-gehemmte LDH₁-Isoenzym nach einem Enzymtest bestimmt wird, umfassend das katalytische Entwickeln eines Chromogens oder einer Farbstoffvorstufe mit dem Isoenzym und im Anschluß daran die Messung der Absorption im Bereich des sichtbaren Lichts oder umfassend die Messung der ultravioletten Absorption des durch die katalytische Aktivität des Isoenzyms reduzierten Coenzyms NADH.

8. Verfahren nach Anspruch 7, das während des gesamten Tests unter neutralen Bedingungen ohne Korrektur des pH-Werts durchgeführt wird.

## Revendications

1. Procédé pour la détermination d'une fraction LDH₁ dans un échantillon, qui comprend l'inhibition de LDH₂, LDH₃, LDH₄ et LDH₅ dans l'échantillon avec une protéase en présence d'un agent dénaturant les protéines puis la détermination de la LDH₁ restant non inhibée.

2. Procédé selon la revendication 1, dans lequel l'échantillon est un échantillon clinique de sérum ou de plasma.

3. Procédé selon la revendication 1 ou 2, dans lequel la protéase est l'α-chymotrypsine, la trypsine, la thrombine, l'élastase, une endoprotéase, la subtilisine, la broméline, la papaïne, la carboxypeptidase, la pronase, la pepsine ou la cathepsine.

4. Procédé selon la revendication 3, dans lequel l'α-chymotrypsine est utilisée comme protéase en une concentration située dans la plage de 10 à 1 000 unités/ml.

5. Procédé selon l'une quelconque des revendications 1 à 4, dans lequel l'agent dénaturant les protéines est l'acide cholique, l'acide désoxycholique, l'acide taurocholique, l'acide taurodésoxycholique, la guanidine (chlorhydrate), l'acide trichloracétique, l'urée, la thiourée, un thiocyanate ou un mélange de ceux-ci.

6. Procédé selon la revendication 5, dans lequel de la guanidine 0,05 à 5 M est utilisée comme agent dénaturant les protéines.

7. Procédé selon l'une quelconque des revendications 1 à 6, dans lequel l'iso-enzyme LDH₁ restant non inhibée est déterminée selon une analyse d'enzyme qui comprend le développement catalytique d'un chromogène ou d'un précurseur de colorant avec l'iso-enzyme puis la mesure de l'absorption dans une plage de lumière visible ou qui comprend la mesure de l'absorption ultraviolette du coenzyme NADH réduit par l'activité catalytique de l'iso-enzyme.

8. Procédé selon la revendication 7, qui est mis en oeuvre dans des conditions neutres au cours de l'analyse sans correction du pH.
